# EUROPEAN PATENT APPLICATION

(11) **EP 2 458 373 A1**
(43) Date of publication of application: **30.05.2012**
(21) Application number: 10802341.7
(22) Date of filing: 23.07.2010
(51) Int. Cl.: G01N 27/30, C12M 1/34, G01N 27/28, G01N 27/416

(54) **CONTAINER FOR MEASURING CELL POTENTIAL AND METHOD FOR PRODUCING SAME**

(30) Priority: 24.07.2009 JP 2009173016
(71) Applicant: NIPRO CORPORATION, Kita-ku Osaka-shi, Osaka 531-8510 (JP)
(72) Inventor: MORIMOTO, Shinji, Osaka-shi Osaka 531-8510 (JP); HAGIWARA, Yui, Osaka-shi Osaka 531-8510 (JP)
(74) Representative: Bertsch, Florian Oliver
(86) International application number: PCT/JP2010/062422
(87) International publication number: WO 2011/010721

(57) **Abstract**

An object of the present invention is to provide a cellular electric potential measuring container with which no wavy baseline occurs and an accurate measurement result can be readily obtained in cellular electric potential measurement using a cellular electric potential measuring container. The present invention provides a cellular electric potential measuring container comprising a container body and an electrode substrate, the electrode substrate being attached to a lower end of the container body so as to form a plurality of wells, the cellular electric potential measuring container being for measuring cellular electric potential after being mounted on an electric potential measuring device, wherein the container body comprises: a plurality of tubular portions whose upper and lower ends are open, and a portion reserved for attachment at the lower end, the electrode substrate comprises a substrate body, with a plurality of measurement electrodes and a plurality of reference electrodes being disposed on one surface of the substrate body, and the container body is attached by a double-sided tape to the surface of the substrate body on which the measurement electrodes and the reference electrodes are disposed, at a place of the portion reserved for attachment, and a method for producing a cellular electric potential measuring container.

## Description

### Technical Field

The present invention relates to a cellular electric potential measuring container for measuring cellular electric potential after being mounted on an electric potential measuring device, and a production method therefor.

### Background Art

In current new drug development, it is necessary to discover the toxicity caused by a drug at an early stage. One known example of this toxicity is drug-induced (acquired) QT prolongation syndrome, which is a disease that causes severe arrhythmia in a patient.

Drug-induced QT prolongation syndrome is a serious disease with which QT interval prolongation appears on an electrocardiogram after drug administration, and ventricular fibrillation often occurs after TdP (Torsades de pointes: non-sustained polymorphic ventricular tachycardia), resulting in syncope or sudden death. In fact, out of the 25 drugs whose sales were stopped in the US market after 1980, five drugs have been determined as causing drug-induced QT prolongation syndrome.

In this regard, to discover toxicity that is caused by a drug, non-patent literature 1 discloses a measurement method in which the effect of a drug on the activity of an ion channel is analyzed based on the change in the electric potential of a cell in a drug-administered culture solution. This measurement method is carried out once a cellular electric potential measuring container is mounted on an electric potential measuring device. This cellular electric potential measuring container includes a plurality of wells for accommodating a culture solution and cells, and a measurement electrode and a reference electrode are disposed on the bottom of each well.

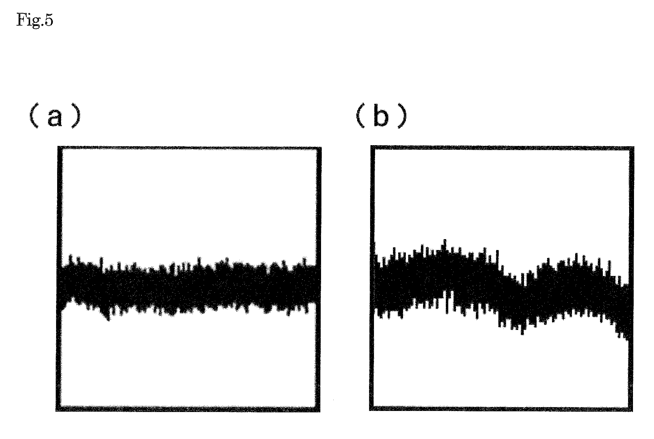

However, with the cellular electric potential measuring container formed by adherence using a liquid adhesive, a wavy baseline in measurement data sometimes occurs as shown in Fig. 5 (b), and thus it is sometimes difficult to obtain accurate measurement results.

### Prior Art Documents

### Non-Patent Documents

Non-Patent Document 1: URL: http://www.brck.co.jp/MCS/qtscreencataloguejp1.pdf

### Summary of Invention

### Problems to be Solved by the Invention

An object of the present invention is to provide a cellular electric potential measuring container with which no wavy baseline occurs and an accurate measurement result can be readily obtained in cellular electric potential measurement using a cellular electric potential measuring container.

### Means for Solving the Problems

To achieve the above-described object, the present invention provides a cellular electric potential measuring container comprising a container body and an electrode substrate, the electrode substrate being attached to a lower end of the container body so as to form a plurality of wells, the cellular electric potential measuring container being for measuring cellular electric potential after being mounted on an electric potential measuring device, wherein the container body comprises: a plurality of tubular portions whose upper and lower ends are open, and a portion reserved for attachment at the lower end, the electrode substrate comprises a substrate body, with a plurality of measurement electrodes and a plurality of reference electrodes being disposed on one surface of the substrate body, and the container body is attached by a double-sided tape to the surface of the substrate body on which the measurement electrodes and the reference electrodes are disposed, at a place of the portion reserved for attachment, and a method for producing a cellular electric potential measuring container.

The cellular electric potential measuring container of the present invention is a disposable component that is used in a system for measuring a change of cellular electric potential that occurs in response to a plurality of new drug candidate compounds in wells. The cellular electric potential measuring container of the present invention is used after being mounted on a dedicated electric potential measuring device (not shown in figure). The configuration thereof includes a plurality of wells. The wells are formed by attaching an electrode substrate to the lower end of a container body. That is, the container body serves as the side wall of the wells, and the electrode substrate serves as the bottom of the wells. The formed wells are fluid tight such that a medium used in a measurement (usually a culture solution) can be accommodated. Both a measurement electrode and a reference electrode are disposed on the bottom of each well. Thereby, the cellular electric potential can be measured in each well. The term "cell" as used herein should be understood to encompass not only a single cell but also cell mass (spheroid) formed by aggregation of a plurality of cells.

The cellular electric potential measuring container of the present invention is disposable as stated above. Therefore, to attain an inexpensive product, it is desirable that at least the container body is entirely made from resin. The resin is not particularly limited in the present invention as long as the resin can be molded into the container body and is electrically insulating. Specific examples of the resin include polypropylene, polystyrene, polyester, polycarbonate, and the like. In particular, from the viewpoint of low material cost, high transparency, and good appearance, the resin is preferably polystyrene. A method for producing the container body is not necessarily limited as long as the container body is readily produced, but given the complex structure of the container body, it is usually desirable to produce the container body by injection molding.

The container body includes tubular portions that serve as the side wall of each well, and a portion reserved for attachment.

The upper and lower ends of the tubular portions are open. The upper end is open to accommodate a cell and a culture solution when carrying out a measurement, and the lower end is open to allow the electrode substrate, which will be described later, to serve as a bottom. The shape of the tubular portions is not particularly limited in the present invention, but it is desirably cylindrical from the viewpoint of easy production.

The portion reserved for attachment is disposed at the lower end of the container body. The portion reserved for attachment is not only a place where a double-sided tape, which will be described later, attaches but also has a role for retaining a plurality of tubular portions. It is desirable that all of the places other than the lower end of the tubular portions are the portions reserved for attachment on the undersurface of the container body to elevate the attachment strength of by the double-sided tape. However, the present invention is not necessarily limited by the areas of the portions reserved for attachment.

Meanwhile, the electrode substrate includes a substrate body, and a plurality of measurement electrodes and a plurality of reference electrodes are disposed on one surface of the substrate body.

The substrate body is composed of a, so-called, electrically insulating material. Examples of the material include polypropylene, polystyrene, polyester, fluororesin, polycarbonate, acrylic resin, paper phenol, paper epoxy, glass composites such as glass epoxy, alumina, and the like. From the viewpoint of easy construction of the measurement electrodes and the reference electrodes, high conductivity, high mechanical strength, and low cost, in general, glass epoxy is often selected. However, the present invention is not limited by these materials for the substrate body

The measurement electrode is an electrode that comes into contact with a cell and measures the ion channel activity of the cell as electric potential, and the reference electrode is an electrode that comes into contact with a medium used in a measurement (usually a culture solution) and measures electric potential that is regarded as, so-called, reference electric potential. The measurement electrode and the reference electrode are disposed in places such that one measurement electrode and one reference electrode are present on the bottom of each well when the electrode substrate is attached to the container body and wells are thus formed. As a matter of course, the measurement electrode and the reference electrode on the bottom of each well are not electrically connected. Examples of materials for the Measurement electrode and the reference electrode include gold, silver, carbon, platinum, ruthenium oxide, palladium, and the like. In particular, gold is suitably used from the viewpoint of high electric conductivity, but the present invention is not limited by these electrode materials. The measurement electrode and the reference electrode can be suitably constructed according to a technique such as screen printing, ink jet, sputtering, or vapor deposition. Therefore, the present invention is also not limited by these electrode constructing methods.

Moreover, the container body is attached to the electrode substrate by a double-sided tape. A place for attachment is a portion reserved for attachment in the container body. Attachment is performed such that no double-sided tape is present at least at the lower end of the tubular portions of the container body. That is, the shape of the double-sided tape is adjusted to the shape of the portion reserved for attachment of the container body. The type of the double-sided tape is not particularly limited as long as the strength of the attachment of the container body and the electrode substrate by the double-sided tape is sufficient, and the double-sided tape has an electrically insulating property, water resistance (water repellent), and chemical resistance. For example, double-sided tapes such as Nichiban double-sided tape (manufactured by Nichiban Co., Ltd.), structural attachment tape (manufactured by Sumitomo 3M Ltd.) are preferably used. Further, attachment is performed such that one surface of the double-sided tape may be attached to the portion reserved for attachment of the container body, and then the other surface of the double-sided tape may be attached to the electrode substrate, or the reverse order is possible. Furthermore, in order to perform accurate attachment, for example, it is desirable to provide the container body, the electrode substrate, and the double-sided tape with a hole for positioning, respectively, however, the present invention is not necessarily limited by the presence or absence of the hole for positioning. Surprisingly, the use of this double-sided tape has removed the wavy baseline in cellular electric potential measurement.

### Effects of Invention

According to the cellular electric potential measuring container of the present invention, it is easy to obtain an accurate measurement result without generating a wavy baseline in measurement data.

### Brief Description of Drawings

[Fig. 1] Fig. 1 is an exploded perspective view of the cellular electric potential measuring container of the present invention.
[Fig. 2] Fig. 2 is a partial bottom view of the container body (for 9 wells) of the cellular electric potential measuring container of the present invention.
[Fig. 3] Fig. 3 is a cross-sectional view of one well of the cellular electric potential measuring container of the present invention (cross section taken along the X-X' in Fig. 4).
[Fig. 4] Fig. 4 is a top view of one well of the cellular electric potential measuring container of the present invention.
[Fig. 5] Fig. 5 (a) is a figure showing a measurement result of cellular electric potential using the cellular electric potential measuring container of the present invention, and Fig. 5 (b) is a figure showing a measurement result of cellular electric potential using the cellular electric potential measuring container formed by adherence using a liquid adhesive.

### Mode for Carrying Out the Invention

Hereinbelow, embodiments of the present invention will be described using the figures, but the present invention is not construed as being limited to the embodiments described later.

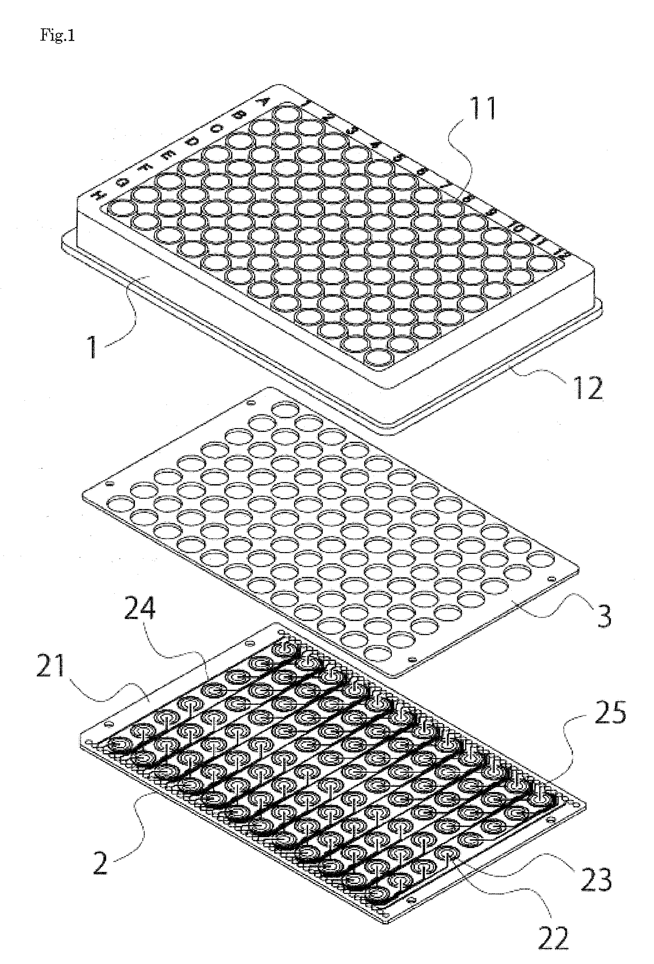

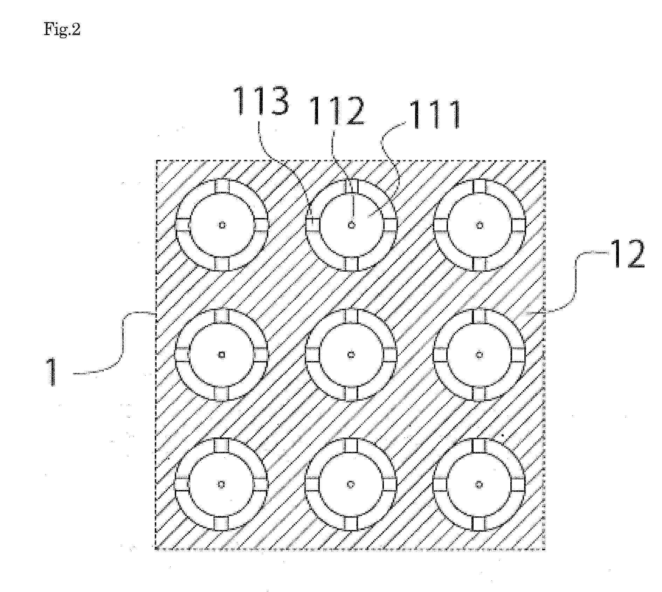

Fig. 1 is an exploded perspective view of the cellular electric potential measuring container of the present invention. The cellular electric potential measuring container of the present invention is primarily composed of a container body 1, an electrode substrate 2, and a double-sided tape 3. The container body 1, which is made from resin, includes a plurality of tubular portions 11 having a cylindrical shape whose upper and lower ends are open, and a portion reserved for attachment 12. The structure of the inner cavity of each tubular portion 11 will be described later. Each tubular portion 11 constitutes the side wall of a well. The portion reserved for attachment 12 is disposed at the lower end of the container body 1. The portion reserved for attachment 12 is also described with reference to Fig. 2. Fig. 2 is a partial bottom view of the container body 1 (for 9 wells). Hatched area is the portion reserved for attachment 12 and also a portion to which the double-sided tape 3, which will be described later, is attached.

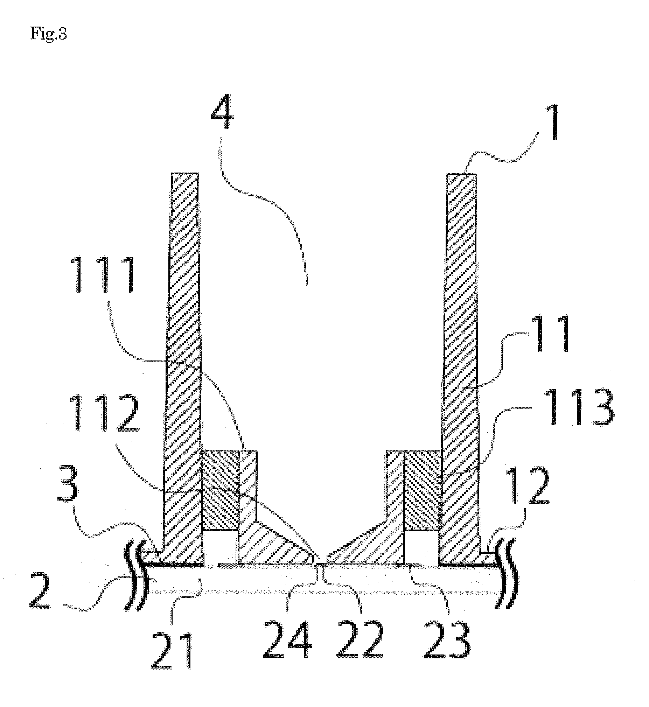

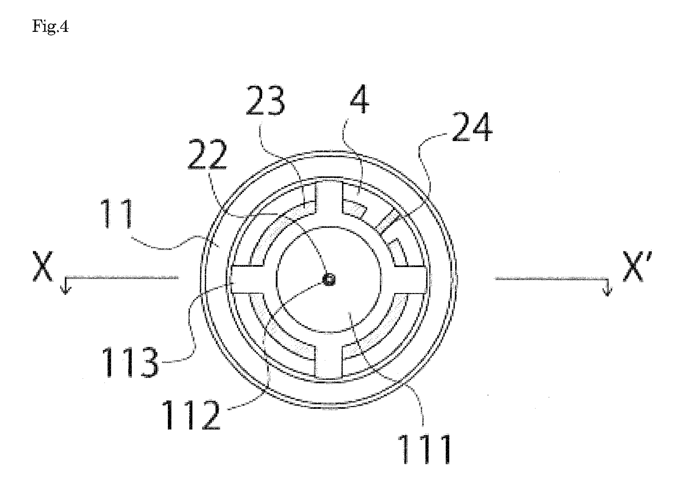

Meanwhile, the electrode substrate 2 forms the bottom of the wells. The electrode substrate 2 includes a substrate body 21 composed of an electrically insulating material, and a plurality of measurement electrodes 22 and reference electrodes 23 are disposed on the substrate body 21. A measurement electrode 22 of the electrode substrate 2 in Fig. 1 is disposed substantially at the center of a reference electrode 23 that has a C shape. The measurement electrode 22 has such a fine structure that it is not clearly visible in Fig. 1, and thus a detailed description is given in reference to Figs. 3 and 4. All of the measurement electrodes 22 and reference electrodes 23 are lead by lead wires 24 that are independent of each other to connectors 25 arranged in line along one side of the electrode substrate. A reason that the reference electrodes 23 are C-shaped is to secure space for disposing lead wires to lead the measurement electrodes 22 to the connectors 25. Although not shown, the connectors 25 are also disposed on the opposite surface of the electrode substrate 2 (the surface on which no measurement electrodes 22 or reference electrodes 23 are disposed). When the cellular electric potential measuring container of the present invention is mounted on a dedicated electric potential measuring device (not shown), the connectors 25 on the opposite surface of the electrode substrate 2 are electrically connected to the dedicated electric potential measuring device, thus enabling cellular electric potential to be measured.

The shape of the double-sided tape 3 is adjusted to the shape of the portion reserved for attachment 12 of the container body 1. That is, the portions corresponding to the open lower end of the tubular portions 11 of the container body 1 is cut off from the double-sided tape 3. This allows the electrode substrate 2 to be exposed as the bottom of the wells. This is readily understood with reference to Fig. 3. Fig. 3 is a cross-sectional view of one well. Since the measurement portion 111, the measurement hole 112, and the retaining means 113 in Fig. 3 are readily understood also with reference to Fig. 4, they will be described later.

Fig. 4 is a preferable embodiment of the cellular electric potential measuring container of the present invention, and a top view of one well 4. For the well 4, the tubular portion 11 of the container body 1 serves as a side wall, and the electrode substrate 2 serves as a bottom. The measurement electrode 22 and the reference electrode 23 are disposed on the bottom of each well 4. The tubular portion 11 that serves as the side wall of the well 4 is provided with a measurement portion 111 having a circular measurement hole 112 and four retaining means 113. The measurement electrode 22 having a fine structure is exposed through the measurement hole 112. This structure allows a cell placed in the measurement portion 111 to move to the measurement hole 112 by its own weight and to come into contact with the measurement electrode 22 exposed through the measurement hole 112. On the other hand, the reference electrode 23 is exposed at the periphery of the measuring portion 111 on the bottom of the well 4.

Fig. 5 (a) shows a measurement result of electric potential (baseline) using the cellular electric potential measuring container of the present invention (a structural attachment tape (manufactured by Sumitomo 3M Ltd.) was used as the double-sided tape 3). For comparison, Fig. 5 (b) shows a measurement result of electric potential (baseline) using the cellular electric potential measuring container formed by adherence using a liquid adhesive Loctite 401 (manufactured by Henkel AG & Co. KGaA) instead of the double-sided tape 3. The result for the cellular electric potential measuring container formed by adherence using a liquid adhesive shows that a wavy baseline occurred. In contrast, the result for the cellular electric potential measuring container of the present invention shows that the wavy baseline was perfectly removed.

A method for measuring cellular electric potential using the cellular electric potential measuring container of the present invention is performed in a manner nearly comparable to a method that uses a conventional cellular electric potential measuring container. However, in the case where the cellular electric potential measuring container of the present invention is configured to include the measurement portion 111 as shown in Figs. 3 and 4, the method is different from conventional methods in that a cell is placed in the measurement portion 111. A cell placed in the measurement portion 111 moves to the measurement hole 112 due to the tapered structure of the measurement portion 111 and by its own weight and can come into contact with the measurement electrode 22 exposed through the measurement hole 112.

### Industrial Applicability

The present invention enables an accurate measurement of cellular electric potential, and therefore the present invention achieves rapid drug screening and will contribute to new drug development.

### Description of Numerals

- 1: Container body
- 11: Tubular portion
- 111: Measurement portion
- 112: Measurement hole
- 113: Retaining means
- 12: Portion reserved for attachment
- 2: Electrode substrate
- 21: Substrate body
- 22: Measurement electrode
- 23: Reference electrode
- 24: Lead wire
- 25: Connector
- 3: Double-sided tape
- 4: Well

## Claims

1. A cellular electric potential measuring container comprising a container body and an electrode substrate, the electrode substrate being attached to a lower end of the container body so as to form a plurality of wells, the cellular electric potential measuring container being for measuring cellular electric potential after being mounted on an electric potential measuring device, wherein
the container body comprises:
a plurality of tubular portions whose upper and lower ends are open, and
a portion reserved for attachment at the lower end,
the electrode substrate comprises a substrate body, with a plurality of measurement electrodes and a plurality of reference electrodes being disposed on one surface of the substrate body, and
the container body is attached by a double-sided tape to the surface of the substrate body on which the measurement electrodes and the reference electrodes are disposed, at a place of the portion reserved for attachment.

2. A method for producing a cellular electric potential measuring container comprising a container body and an electrode substrate, the electrode substrate being attached to a lower end of the container body so as to form a plurality of wells, the cellular electric potential measuring container being for measuring cellular electric potential after being mounted on an electric potential measuring device, wherein
the container body comprises:
a plurality of tubular portions whose upper and lower ends are open, and
a portion reserved for attachment at the lower end,
the electrode substrate comprises a substrate body, with a plurality of measurement electrodes and a plurality of reference electrodes being disposed on one surface of the substrate body, and
the method comprises attaching by a double-sided tape the container body to the surface of the substrate body on which the measurement electrodes and the reference electrodes are disposed, at a place of the portion reserved for attachment.
